# EUROPÄISCHE PATENTSCHRIFT

(11) **EP 1 235 901 B1**
(45) Veröffentlichungstag und Bekanntmachung des Hinweises auf die Patenterteilung: **24.08.2011**
(21) Anmeldenummer: 00991145.4
(22) Anmeldetag: 06.12.2000
(51) Int. Cl.: C12N 5/00, A61L 27/00, A61L 27/38, A61P 17/00

(54) **VERFAHREN ZUR HERSTELLUNG VON ZELLTRANSPLANTATEN**
METHOD FOR PRODUCING CELL TRANSPLANTS
PROCEDE POUR PREPARER DES TRANSPLANTS CELLULAIRES

(30) Priorität: 06.12.1999 DE 19958693
(43) Veröffentlichungstag der Anmeldung: 04.09.2002
(73) Patentinhaber: Biotissue Technologies GmbH, 79108 Freiburg (DE)
(72) Erfinder: SEUBERT, Rainer, 79183 Waldkirch/Kollnau (DE); TANCZOS, Eszter, 79104 Freiburg (DE)
(74) Vertreter: Ricker, Mathias
(86) Internationale Anmeldenummer: PCT/EP2000/012290
(87) Internationale Veröffentlichungsnummer: WO 2001/040444

(56) Entgegenhaltungen:
- EP-A- 0 373 044
- US-A- 4 304 866
- KAUFMANN R ET AL: "GRAFTING OF IN VITRO CULTURED MELANOCYTES ONTO LASER-ABLATED LESIONS IN VITILIGO" ACTA DERMATO-VENEREOLOGICA, Bd. 78, Nr. 2, 1998, Seiten 136-138, XP001003022 ISSN: 0001-5555
- ANDREASSI L ET AL: "A NEW MODEL OF EPIDERMAL CULTURE FOR THE SURGICAL TREATMENT OF VITILIGO" INTERNATIONAL JOURNAL OF DERMATOLOGY, Bd. 37, Nr. 8, August 1998 (1998-08), Seiten 595-598, XP001003030 ISSN: 0011-9059
- ZACHARIAE H ET AL: "AUTOTRANSPLANTATION IN VITILIGO: TREATMENT WITH EPIDERMAL GRAFTS AND CULTURED MELANOCYTES" ACTA DERMATO-VENEREOLOGICA, Bd. 73, 1993, Seiten 46-48, XP001001501 ISSN: 0001-5555
- PELLEGRINI G ET AL: "CULTIVATION OF HUMAN KERATINOCYTE STEM CELLS: CURRENT AND FUTURE CLINICAL APPLICATIONS" MEDICAL AND BIOLOGICAL ENGINEERING AND COMPUTING,GB,PETER PEREGRINUS LTD. STEVENAGE, Bd. 36, Nr. 6, 1. November 1998 (1998-11-01), Seiten 778-790, XP000784853 ISSN: 0140-0118

## Beschreibung

Die vorliegende Erfindung betrifft allgemein die Gebiete der eukaryotischen Zellkultur und des *Tissue-Engineering.* Die vorliegende Erfindung betrifft ein Verfahren zur Herstellung eines humanen autologen Transplantats.

Der medizinisch-biotechnologische Arbeitsbereich des *Tissue Engineering* umfaßt die Entwicklung, Herstellung und Modifizierung von Implantaten, *in vitro* hergestellten Biomolekülen, Zellen oder Geweben zum Ersatz oder zur Unterstützung der Funktion fehlender oder verletzter Körperteile und Gewebe. Ein Schwerpunkt liegt hierbei im Bereich der Herstellung sogenannte autologer Transplantate. Das zugrunde liegende Prinzip besteht darin, daß einem Patienten Zellen des zu transplantierenden Typs aus einem gesunden Gewebebereich, dem sog. Spenderareal, entnommen, *in vitro* kultiviert, zur Herstellung eines Transplantats, beispielsweise durch Einbringen in eine geeignete Trägermatrix, verwendet und schließlich auf ein entsprechend vorbereitetes Empfängerareal desselben Patienten aufgebracht werden. Ein entscheidender Vorteil dieser Art von Therapie liegt darin, daß Abstoßungsreaktionen sowie Infektionsübertragung, wie sie bei heterologen Transplantationen häufig auftreten, in aller Regel nicht zu befürchten sind. Prinzipiell ist die Herstellung derartiger autologer Transplantate auf der Basis einer Vielzahl von Zell- und Gewebetypen denkbar, beispielsweise Haut-, Knorpel-, Knochen-, Fett- oder andere Gewebetypen. In der Praxis bietet sich etwa insbesondere die Haut aufgrund ihrer exponierten Lage und somit leichten Zugänglichkeit an. Zudem gibt es verschiedene dermatologische Indikationsgebiete, die eine - häufig großflächige - Hauttransplantation erfordern. Hierbei sind zum einen insbesondere schwere Brandverletzungen sowie schwer heilende Hautgeschwüre, zum anderen Pigmentstörungen wie etwa Vitiligo ("Weißfleckenkrankheit") zu nennen.

Vitiligo ist eine erworbene idiopathische, hypomelanotische Hautveränderung, die durch den Verlust von Melanozyten in Haut und Haarbulbus charakterisiert ist. Typische klinische Symptome sind scharf begrenzte depigmentierte Makulae (Flecken) unterschiedlicher Größe, Form und Ausdehnung, die an jeder Körperstelle lokalisiert sein können. Die Prävalenz der Vitiligo liegt etwa zwischen 0,5 bis 4%. Am häufigsten tritt die Erkrankung bei jungen Leuten auf.

Trotz intensiver Forschung bleibt die Vitiligo eine Pigmentstörung unbekannter Ätiopathogenese. Dementsprechend ist eine kausale Behandlung der Vitiligo bisher noch nicht möglich. Die gegenwärtigen Therapieansätze beschränken sich daher darauf, eine Repigmentation zu erzielen. Hierbei unterscheidet man zwischen operativen und nicht-operativen Repigmentationsmaßnahmen. Adjuvante Behandlungen wie Camouflage oder psychologische Beratung haben sich als sinnvoll erwiesen, da die mit Vitiligo verbundene kosmetische Endstellung häufig psychosoziale Probleme der Betroffenen bedingt.

Gängige nicht-operative Maßnahmen bei der Vitiligo-Therapie sind etwa die Kombinationstherapie mit Psoralen und UVA-Bestrahlung, die sogenannte PUVA-Therapie, die UVB-Bestrahlungstherapie, sowie die lokale bzw. systemische Kortikosteroidtherapie, ggf. ebenfalls in Verbindung mit einer UVA-Bestrahlung. Nachteile dieser konventionellen nicht-operativen Repigmentationsmaßnahmen liegen vor allem darin, daß Patienten auf die PUVA-Therapie in der Regel erst nach 30-40 Behandlungen und dann in sehr unterschiedlichem Maße ansprechen, sowie in UVA-bedingten phototoxischen Reaktionen sowie in den üblichen mit der Kortikosteroidtherapie verbundenen Nebenwirkungen.

Operative Maßnahmen zur Behandlung der Vitiligo sind oft die Therapie der Wahl, da die konservativen Therapiemaßnahmen sehr oft erfolglos bleiben; sie können gegebenenfalls in Kombination mit einer medikamentösen und/oder einer Bestrahlungstherapie angewandt werden. Als operative Repigmentationsmaßnahmen sind allgemein die Mikrotätowierung sowie verschiedene autologe Transplantationsmethoden zu nennen. Bei der Mikrotätowierung werden Eisenoxidpigmente läsionell implantiert. Es ist hierbei insbesondere problematisch, eine befriedigende Farbabstimmung mit der umgebenden Haut zu erreichen, nicht zuletzt aufgrund der saisonalen Hautfarbenvariation.

Die in der Praxis viel bedeutsameren autologen Transplantationsmethoden lassen sich im wesentlichen in vier Gruppen einteilen: (1) Transplantation autologer, in der Regel 1-2 mm dicker Punchgrafts von normal pigmentierter Haut in ein depigmentiertes Empfängerareal (*Minigrafting*); (2) Transplantation autologer Spalthauttransplantate; (3) Transplantation autologer *Suction blister*-Transplantate; sowie (4) Transplantation autologer Hautzellen, etwa Melanozyten mit oder ohne Keratinozyten.

Im Zusammenhang mit der Behandlung von Vitiligopatienten mittels autologer Transplantation von Hautzellen offenbart der Stand der Technik sowohl die Verwendung nicht-kultivierter als insbesondere die kultivierter autologer Hautzellen. Die erste Transplantation autologer kultivierter Melanozyten in einem Vitiligopatienten wurde in 1987 von Lerner et al. (J. Invest. Dermatol., 1987, Vol. 89, Seiten 219-224) durchgeführt.

Gauthier et al. (Journal of the American Academy of Dermatology, 1992, Vol. 26(1), Seiten 191-194) offenbaren, daß sich insbesondere Suspensionen aus nichtkultivierten Melanozyten und Keratinozyten zur Vtiligo-Therapie eignen. Ein entscheidender Nachteil solcher Transplantationen nicht-kultivierter Zellen liegt jedoch darin, daß nur eine geringe Anzahl von Zellen für die Transplantation zur Verfügung steht

Zachariae et al. (Acta Derm. Venereol., 1993, Seiten 46 - 48) berichten über die erfolgreiche Transplantation kultivierter Melanozyten nach der Aussiedlung der Zellen auf einem Kollagenfilm, allerdings bei einer nur sehr geringen, statistisch nicht signifikanten Anzahl von Patienten. Eine Transplantation kultivierter Melanozyten in Verbindung mit einer formbaren oder gelartigen Trägermatrix ist im Stand der Technik mit keinem Wort erwähnt.

Trotz der im Stand der Technik offenbarter Erfolge mit autologen Hautzelltransplantaten liegt der Schwerpunkt der operativen Vitiligotherapiemaßnahmen in der Praxis immer noch bei den autologen Gewebetransplantationen. Dies liegt unter anderem daran, daß sich derartige Methoden in der routinemäßigen Praxis und in einem größeren Maßstab einfacher anwenden lassen, obwohl sie insbesondere unter kosmetischen Gesichtspunkten häufig unerwünschte Nebenwirkungen mit sich bringen. Im Vergleich ist eine Transplantation kultivierter Zellen unter verschiedenen Aspekten vorteilhaft. So ist etwa im Vergleich mit den klassischen chirurgischen Transplantationsmethoden die Infektionsgefahr sowohl in der Spender- als auch in der Empfängerregion stark reduziert, Wundheilungsstörungen treten seltener auf, die Entnahme der zu kultivierenden autologen Zellen sowie deren spätere Transplantation verlaufen nahezu oder vollkommen schmerzfrei, und die Ergebnisse sind auch unter ästhetischen Gesichtspunkten viel befriedigender. So werden durch die Transplantation kultivierter Zellen verschiedene unerwünschte Begleiterscheinungen der klassischen chirurgischen Transplantationsmethoden, etwa der "Pflastersteineffekt" der Empfängerstelle, eine Keloidbildung oder die Bildung hypertropher Narben, vermieden.

Trotz des Wissens um diese Vorteile und des sich daraus ergebenden Bedürfnisses, derartige autologe Zelltransplantationen einer breiteren Anwendung zugänglich zu machen, ist es bisher noch nicht gelungen, ein verfahren zur Herstellung von humanen autologen Transplantaten, zu entwickeln, das sich zur routinemäßigen Durchführung auch im größeren Maßstab eignet und sowohl die erforderliche Sicherheit und Qualität als auch die nötige Effizienz gewährleistet. Insbesondere wurde bisher noch kein derartiges Verfahren zur Herstellung von humanen autologen Transplantaten auf der Basis eukaryotischer menschlicher Zellen entwickelt, welches den Anforderungen des Arzneimittelgesetzes und anderer gesetzlicher Vorschriften wie etwa den relevanten EG-Verordnungen genügt.

Somit bestand eine der gegenwärtigen Erfindung zugrunde liegenden Aufgabe darin, ein Verfahren bereitzustellen, das eine routinemäßige Kultivierung humaner eukaryotischer Zellen, zur Herstellung von autologen, Transplantaten, ermöglicht, das den behördlichen Qualitäts- und Sicherheitsanforderungen, insbesondere dem Arzneimittelgesetz und den EG-Verordnungen für pharmazeutische Hersteller bezüglich einer *good manufacturing practice* (GMP), genügt und somit auch eine Anwendung im größeren und gegebenenfalls auch im kommerziellen Maßstab erlaubt.

Es wurde nun gefunden, daß eine routinemäßige Kultivierung eukaryotischer, humaner Zellen für die Herstellung von autologen (Zell-)Transplantaten, die den behördlichen Qualitäts- und Sicherheitsanforderungen genügt, möglich ist, wenn Bedingungen, die den Anforderungen der Reinheitsklasse A gemäß den GMP-Richtlinien entsprechen, eingehalten werden. Eine Anwendung einer derartigen Reinstraumtechnologie im Bereich des *Tissue Engineering* an sich und insbesondere zur Herstellung humaner Transplantate ist dem jetzigen Anmelder nicht bekannt.

Demgemäß betrifft die vorliegenden Erfindung ein Verfahren zur Herstellung eines humanen autologen Transplantats, dadurch gekennzeichnet, dass (1) humane eukaryotische Zellen so kultiviert werden, dass Schritte, bei denen die Zellkulturen offen sind, in einem geschlossenen System bei Überdruck durchgeführt werden, und dass in dem System (a) pro Kubikmeter Luft nicht mehr als 3500 Partikel vorhanden sind, die mindestens 0,5 µm groß sind; und (b) pro Kubikmeter Luft keine Partikel vorhanden sind, die mindestens 5 µm groß sind; die kultivierten Zellen unter den in (1) genannten Bedingungen in eine geeignete Matrix aufgenommen werden, wobei das Transplantat ein dreidimensionales Transplantat ist, innerhalb dessen die kultivierten autologen Zellen in Verbindung mit einer geeigneten Matrix in eine vorgegebene, dreidimensionale Form gebracht wurden. Das Verfahren gemäß der vorliegenden Erfindung ist insbesondere dadurch charakterisiert, daß die Arbeitsschritte, bei denen die Zellkulturen offen sind, d.h. mit der umgebenden Luft in Berührung kommen, in einem geschlossenen, d.h. einem gegenüber der Umgebung hermetisch abgegrenzten System bei Überdruck durchgeführt werden. Damit unterscheidet sich die Kultivierungsmethode im Rahmen der vorliegenden Erfindung sowohl von den herkömmlichen Verfahren zur Kultivierung eukaryotischer Zellen, bei denen die Arbeitsschritte, bei denen die Zellkulturen offen sind, in einem offenen System bei Überdruck durchgeführt werden, als auch von den geschlossenen Systemen, die etwa im Bereich der Infektionsforschung oder in der Transplantationsmedizin (z.B. im Zusammenhang mit Knochenmarkstransplantationen) angewandt werden, in denen ein Unterdruck herrscht und die einen kompletten Personenschutz erfordern.

Gamäß der vorliegenden Erfindung werden die genannten Herstellungsschritte in Arbeitsisolatoren, die den Anforderungen der Reinheitsklasse A gemäß den GMP-Richtlinien entsprechen, durchgeführt, d.h. unter nahezu keimfreien Bedingungen. Technisch wird diese Keimfreiheit durch mehrere Faktoren gesichert, insbesondere Überdruck in dem Isolator, Laminar Air Flow, sowie durch den Einsatz geeigneter Vorrichtungen, etwa Schwebstofffilter, beispielsweise mittels eines für die Reinheitsklasse A geeigneten HEPA (*High efficiency particular airfiltration*)-Filters, mittels dessen 99,99 % aller Teilchen, die größer als 0,3 µm sind, entfernt werden können, sowie durch desinfiziertes Einschleusen von Materialien, beispielsweise aus einem Reinraum der Klasse D.

Die Spezifikation der Reinheitsklassen im Zusammenhang mit der vorliegenden Erfindung entspricht den zum Zeitpunkt der Anmeldung gültigen Anforderungen gemäß dem EG-Leitfaden einer Guten Herstellungspraxis für Arzneimittel (vgl. etwa "EG-Leitfaden einer Guten Herstellungspraxis für Arzneimittel - mit

Betriebsverordnung für pharmazeutische Unternehmer" (1998), 5., überarbeitete und erweiterte Auflage, Hrsg: G Auterhoff; Editio Cantor Verlag Aulendorf). Darin enthält die erste ergänzende und überarbeitete Leitlinie für die Herstellung steriler Arzneimittel im Anhang dieses EG-Leitfadens Definitionen der Reinheitsklassen A, B, C und D, wobei die quantitative Klassifizierung nach den in der Luft enthaltenen Partikeln erfolgt.

Für die Reinheitsklasse A etwa gilt, daß nicht mehr als 3500 Partikel, die mindestens 0,5 µm groß sind, und 0 Partikel, die mindestens 5 µm groß sind, pro Kubikmeter Luft vorhanden sein dürfen. Die empfohlenen (Durchschnitts-)Grenzwerte für die mikrobiologische Kontaminierung betragen in der Reinheitsklasse A im operationellen Zustand, d.h. dem Zustand, in dem die Anlage in der vorgesehenen Art mit der angegebenen Personalstärke betrieben wird, jeweils < 1 cfu/m³, d.h. sowohl in der Luftprobe; für Petrischalen mit einem Durchmesser von 90 mm über einen Zeitraum von 4 Stunden; für Kontaktplatten mit einem Durchmesser von 55 mm; sowie für einen 5 Finger-Handschuhabdruck (vgl. a.o.O insbesondere Seiten 73 - 77).

In einer bevorzugten Ausführungsform stehen die Isolatoren der Reinheitsklasse A in einer Umgebung der Reinheitsklasse D. Diese Isolatoren bestehen aus festverbundenen Transfer- und Arbeitsisolatoren. Die GMP-Bereiche müssen mit entsprechenden Lüftungssystemen ausgestattet sein. Bevorzugt wird ein etwa 20-flacher Luftwechsel pro Stunde gewährleistet. In dieser bevorzugten Ausführungsform passiert die aufbereitete Luft über Auslässe einen Schwebstofffilter der Klasse S2-HEPA-Filter und wird dann wieder in den GMP-Bereich der Reinheitsklasse D eingeblasen. Wie bereits erwähnt, liegt der Kapazität eines solchen Filters bei mehr als 99,997 % bei einer Partikelgröße von 0,3 um.

Während der eigentlichen Produktionsphase sind die Arbeitsisolatoren nur über Transferisolatoren zu beladen, die im Zusammenhang mit der vorliegenden Erfindung bevorzugt der Reinraumklasse B zuzuordnen sind. Die Transferisolatoren sind ebenfalls mit einem entsprechenden Lüftungssystem ausgerüstet, so daß auch in den Transferisolatoren die Luft ständig über einen Filter ausgewechselt wird. Um eine aseptische Einschleusung von Materialien in die Arbeitsisolatoren zu gewährleisten, ist eine Beladung der Transferisolatoren nur dann möglich, wenn die Arbeitsisolatoren geschlossen sind. Sowohl die Arbeits- als auch die Transferisolatoren werden monatlich mittels Wasserstoffperoxid-Begasung sterilisiert. Der übrige, die genannten Arbeits- und Transferisolatoren umgebende Reinraum kann einer niedrigeren Reinstraumklasse angehören, beispielsweise den Reinraumklassen B, C, oder D. In diesem die Arbeits- und Transferisolatoren umgebenden Reinraum werden gemäß einer bevorzugten Ausführungsform der vorliegenden Erfindung diejenigen Kultivierungsschritte bzw. Schritte zur Herstellung eines autologen oder allogenen (Zell-)Transplantats durchgeführt, bei denen die Zellkulturen nicht offen sind.

In diesem den Isolator umgebenden Reinraumbereich, welcher gemäß einer bevorzugten Ausführungsform der vorliegenden Erfindung der Reinheitsklasse C oder D zuzuordnen ist, ein ständiger Überdruck gegenüber dem normalen atmosphärischen Druck gewährleistet sein. Bevorzugt ist ein Überdruck von größer als etwa 5 - 10 Pascal. Ein noch größeres Druckgefälle besteht zwischen den Arbeitsisolatoren der Reinheitsklasse A und der Umgebung der niedrigeren Reinheitsklasse, bevorzugt wird in dem Arbeitsisolator der Reinheitsklasse A ein entsprechender Überdruck von größer als etwa 25 Pascal, bevorzugt von größer als etwa 50 Pascal eingestellt.

Somit befindet sich zwischen dem Arbeitsisolator, in dem die Kultivierungs- bzw. Herstellungsschritte, in denen die Zellkulturen offen sind, unter Bedingungen der Reinheitsklasse A durchgeführt werden, und der Umgebung, in der Bedingungen niedrigerer Reinheitsklassen, bevorzugt C oder D vorliegen, sowohl eine Druckbarriere als auch eine physikalische, durch die Bauweise des Isolators bedingte Barriere, wobei letztere beispielsweise aus Kunststoffglas besteht.

In einer bevorzugten Ausführungsform der vorliegenden Erfindung bestehen die Arbeits- und Transferisolatoren der Reinheitsklasse B beispielsweise aus Edelstahl mit abgerundeten Innenkanten (Hohlkehle) und stellen Handschuhsysteme dar. Prinzipiell können jedoch natürlich sowohl die Arbeits- und Transferisolatoren also auch die verwendeten Handschuhe aus jedem für den zu erfüllenden Zweck geeigneten Material bestehen.

Selbstverständlich können gemäß der vorliegenden Erfindung auch alle im Rahmen der Herstellung von (Zell-)Transplantaten durchzuführenden Schritte, d.h. sowohl die, bei denen die Zellen "offen" sind, d.h. Kontakt mit der umgebenden Luft haben, als auch diejenigen, bei denen die Zellen nicht in derartigem Kontakt stehen, z.B. während der Inkubationszeiten im Brutschrank oder während des Zentrifugierens, unter Bedingungen der Reinheitsklasse A durchgeführt werden. Die einzige Voraussetzung hierfür ist, daß die erforderlichen Apparaturen etc. in den Reinraum dieser Reinheitsklasse eingeschleust und in Betrieb genommen werden können.

Der erfindungsgemäße Schritt (1) ist prinzipiell zur Kultivierung humaner eukaryotischer Zellen jeglicher Art geeignet. Im Rahmen der vorliegenden Erfindung dient er zur Kultivierung von humanen eukaryotischen Zellen im Rahmen der Herstellung von autologen (Zell-)Transplantaten, Dabei umfaßt die vorliegende Erfindung die entsprechende Kultivierung zur Herstellung dreidimensionaler Transplantate, beispielsweise zur Herstellung von dreidimensionalem Haut-, Knochen- oder Knorpelersatz. Bei dieser Art von Transplantaten kann auch die Form der zu transplantierenden Gewebe vorgegeben werden. Zur Veranschaulichung seien hier beispielhaft lediglich ein dreidimensionaler Gelenkersatz, ein dreidimensionaler Fingerknochenersatz oder ein dimensionaler Ohrersatz genannt.

Im Rahmen der vorliegenden Erfindung werden bevorzugt Hautzellen, insbesondere Melanozyten, Keratinozyten und Fibroblasten, sowie Knochenzellen, Knorpelzellen, Adipozyten, Gefäßzellen, Mundschleimhautzellen, Urothelzellen, Muskelzellen, Nervenzellen, hämatopoetische Zellen, Sehnenzellen, andere Zellen des Bewegungssystems, embryonale oder andere Stammzellen, Leberzellen, Nierenzellen, Herzmuskelzellen, verschiedene Schleimhautepithelzellen und Hormone oder Transmitter produzierende Zellen, beispielsweise Inselzellen des Pankreas, Drüsenzellen, corneale Epithelzellen etc., gemäß dem erfindungsgemäßen Verfahren kultiviert. Besonders bevorzugt werden Hautzellen, insbesondere Melanozyten, Keratinozyten und Fibroblasten, sowie Knochenzellen, Knorpelzellen, Adipozyten, Gefäßzellen, Mundschleimhautzellen, Urothelzellen, Muskelzellen und Nervenzellen gemäß dem erfindungsgemäßen Verfahren kultiviert.

Dabei sind die Parameter und Bedingungen, die bei der Kultivierung humaner eukaryotischer Zellen eingehalten werden müssen (Zusammensetzung der Kulturmedien, Temperatur, CO₂-Gehalt, etc.), dem Fachmann grundsätzlich bekannt, ebenso zusätzliche Methoden, die sich als besonders vorteilhaft bei der Kultivierung humaner eukaryotischer Zellen erwiesen haben. Beispielhaft seien hier etwa Spinnerkulturtechniken genannt, bei denen die Zellen auf sogenannten Microcarriern, d.h. kleine Kügelchen bestehend aus geeigneten, beispielsweise resorbierbaren biologischen oder synthetischen, Materialien, kultiviert werden, wodurch eine große Oberflächenexpansion erzielt wird.

Wie bereits erwähnt, lassen sich gemäß dem erfindungsgemäßen Verfahren autologe, humane (Zell-)Transplantate, herstellen. Wie nachfolgend noch detaillierter erläutert werden wird, können diese (Zell-)Transplantate gegebenenfalls geeignete Trägersubstanzen enthalten.

Wie oben bereits erwähnt, ist es prinzipiell in vielen Fällen möglich, einem Gewebe entnommene humane eukaryotische Zellen zu kultivieren und zur Herstellung autologer, Transplantate zu verwenden, beispielsweise für die Behandlung von Gewebedefekten oder bei Organversagen.

Die prinzipielle Vorgehensweise sei anhand der folgenden zwei Beispiele kurz dargestellt, die nachfolgend noch etwas detaillierter erläutert werden werden:
- (i): Herstellung eines Gelenkersatzes beinhaltend die Entnahme eines Knorpelstücks, die Kultivierung der Knorpelzellen und die Verbindung mit geeigneten synthetischen oder biologischen Matrixmaterialien;
- (ii): Herstellung eines Knochenstücks beinhaltend die Entnahme eines Knochenstückes oder einer Knochenmarkbiopsie, die Kultivierung der Knochenzellen (beispielsweise Knochenvorläuferzellen oder gereifte Knochenzellen) und die Verbindung mit geeigneten synthetischen oder biologischen Matrixmaterialien.

Aus dem Gesagten wird klar, daß eine derartige Vorgehensweise prinzipiell bei allen eukaryotischen bzw. menschlichen Zelltypen vorstellbar ist.

In einer bevorzugten Ausführungsform werden gemäß dem erfindungsgemäßen Verfahren humane Melanozyten und Keratinozyten, gegebenenfalls in Verbindung mit Fibroblasten und/oder anderen (Haut-)Zellen, sowie gegebenenfalls auch im Rahmen einer dreidimensionalen Hautkultur, zum Zwecke der Herstellung autologer humane Transplantate kultiviert. Besonders bevorzugt beinhaltet die Herstellung solcher Melanozyten- und/oder Keratinozytentransplantate unter Verwendung des erfindungsgemäßen Verfahrens nach oder während der Kultivierung der entsprechenden Hautzellen einen Schritt, in dem die Zellen in eine geeignete Trägermatrix eingebracht werden. Dies wird im folgenden noch detaillierte erläutert werden.

In derartigen auf Hautzellen basierenden Zellkulturen bzw. Transplantaten ist das Verhältnis zwischen Melanozyten und weiteren vorhandenen Zelltypen, beispielsweise Keratinozyten, unkritisch; ebenso ist ein alleiniges Vorliegen eines Zelltyps, etwa von Melanozyten bzw. Keratinozyten denkbar und möglich.

Wie erwähnt, zeichnet sich das erfindungsgemäße Verfahren insbesondere dadurch aus, daß es den höchsten Sicherheitsstandards genügt und somit für die Herstellung autologer (Zell-)Transplantate geeignet ist. Damit ist dem Fachmann erstmals eine Methode an die Hand gegeben, mittels derer er die Herstellung derartiger Transplantate routinemäßig und im größeren Maßstab unter Einhaltung der behördlichen Sicherheits- und Qualitätsanforderungen durchführen kann.

Das erfindungsgemäße Verfahren kann prinzipiell zur Herstellung aller autologen Transplantate, die auf kultivierten humanen eukaryotischen Zellen basieren, verwendet werden. Bevorzugt wird das erfindungsgemäße Verfahren zur Herstellung autologer Transplantaten verwendet, welche Hautzellen, hierbei insbesondere Melanozyten, Keratinozyten und/oder Fibroblasten, Knochenzellen, Knorpelzellen, Fettzellen, Mundschleimhautzellen, Gefäßzellen, Nervenzellen, Muskelzellen oder Urothelzellen enthalten.

Die vorliegende Erfindung betrifft ferner ein Verfahren zur Herstellung von humanen autologen Transplantaten, nämlich Transplantate enthaltend Melanozyten und/oder Keratinozyten, dadurch gekennzeichnet, daß die Melanozyten und/oder Keratinozyten gemäß dem erfindungsgemäßen Verfahren kultiviert wurden.

Gemäß dem erfindungsgemäßen Verfahren werden die zu transplantierenden humanen eukaryotischen Zellen nach der Entnahme aus dem Spendergewebe unter den oben definierten Bedingungen (Reinheitsklasse A zumindest bei Kultivierungs- bzw. Herstellungsschritten, in denen die Zellkulturen offen sind) kultiviert und nachfolgend - wenn erwünscht - unter eben solchen Bedingungen in eine geeignete Matrix aufgenommen oder anderweitig in eine für die Transplantation geeignete Form gebracht oder für eine Verwendung im Rahmen einer Behandlung eines oder mehrerer Gewebedefekte oder eines Organvarsagen vorbereitet.

In diesem Zusammenhang lassen sich beispielhaft etwa nennen: Transplantate aus suspendierten Zellen, in denen die Zellen bevorzugt in einem gelartigen, spritzbaren Material vorliegen; Zellrasen, d.h. einschichtige zelluläre Strukturen, die auf einem geeigneten, beispielsweise membranartigen Trägermaterial aufgebracht sind; sowie dreidimensionale Transplantate, innerhalb derer die kultivierten autologen Zellen in Verbindung mit einer geeigneten Matrix in eine vorgegebene, dreidimensionale Form gebracht werden, beispielsweise die eines Ohres oder eines Gelenks.

Insbesondere bei der Herstellung autologer Transplantate, die Hautzellen, insbesondere Melanozyten und/oder Keratinozyten, Knochen- und/oder Knorpelzellen, gegebenenfalls in Verbindung mit weiteren Zellen, enthalten, wurde festgestellt, daß es häufig von Vorteil ist, wenn die Zellen in Verbindung mit einer geeigneten Matrix als Trägermaterial transplantiert werden. Dementsprechend werden derartige Zellen häufig während oder nach der Kultivierung, aber vor der Transplantation in ein entsprechendes Trägermaterial aufgenommen.

Eine für diesen Zweck geeignete Matrix kann prinzipiell entweder auf biologischen Molekülen basieren, die *in vivo* beispielsweise als Bestandteile der extrazellulären Matrix (ECM) vorkommen, etwa Kollagene, Hyaluronsäureester und Glykosaminoglykansäureester, oder auf Derivaten solcher ECM-Moleküle, des weiteren auf Fibrin oder Fibrinderivaten sowie auf geeigneten synthetischen Trägermaterialien. In der Regel werden biologisch abbaubare Materialien bevorzugt.

Bei dem erfindungsgemäßen Verfahren zur Herstellung autologer, Melanozyten und/oder Keratinozyten enthaltender Transplantate wird bevorzugt eine Trägermatrix eingesetzt, die im folgenden näher erläutert werden wird und die insbesondere dadurch gekennzeichnet ist, daß sie eine formbare oder gelartige Konsistenz aufweist.

Ein besonderer Vorteil dieser formbaren bzw. gelartigen Konsistenz ist darin zu sehen, daß die entsprechenden Transplantate besonders für eine Transplantation auf problematische Empfängerareale etwa an den Händen, am Nacken, am Hals oder im Genitalbereich geeignet sind, d.h. auf die Areale, die in der Regel am stärksten von der Vitiligo betroffen sind.

Bevorzugt wird demgemäß bei dem erfindungsgemäßen Verfahren zur Herstellung autologer, Melanozyten und/oder Keratinozyten enthaltender Transplantate eine Trägermatrix eingesetzt, die die Kollagengel, Fibrinkleber oder ein oder mehrere andere Fibrinderivate, Hyaluronsäurederivate oder Hydrogele oder Mischungen davon enthält, besonders bevorzugt ist eine Fibrinkleber und/oder ein oder mehrere (andere) Fibrinderivate enthaltende Trägermatrix.

Im Zusammenhang mit Knochentransplantaten ist insbesondere der Einsatz einer humanen Spongiosa geeignet, d.h. einer autoklavierten oder mittels anderer Verfahren desinfizierten Knochensubstanz von menschlichen Knochen, die allerdings keine Knochenzellen mehr enthält. Die besten Ergebnisse lassen sich erzielen, wenn Knochenzellen in Gegenwart einer humanen Spongiosa kultiviert oder im Anschluß an die Kultivierung mit einer humanen Spongiosa zusammengebracht werden. Prinzipiell sind auch andere Trägermatrizes in Verbindung mit Knochentransplantaten denkbar, etwa Biomoleküle, die Bestandteile der extrazellulären Matrix darstellen, oder auch geeignete synthetische Materialien, bevorzugt sind hierbei jedoch solche Moleküle und/oder Materialien, die eine poröse Struktur aufweisen. Beispielhaft wären an dieser Stelle etwa Hydroxyapatit, Calciumphosphat sowie Kollagene zu nennen. Des weiteren ist ein spritzbares Knochentransplantat, bevorzugt auf der Basis einer formbaren oder gelartigen Matrix, etwa auf der Basis von Fibrin, ebenfalls vorstellbar.

Auch im Zusammenhang mit der Herstellung autologer Knorpeltransplantate gemäß der vorliegenden Erfindung ist dem Fachmann bei der Auswahl geeigneter Trägermaterialien keinerlei Beschränkungen ausgesetzt. Bevorzugt werden im Rahmen der vorliegenden Erfindung auch in Verbindung mit Knorpelzellen (Chondrozyten) gelartige und/oder spritzbare Formen von Transplantaten bevorzugt, die Trägersubstanz enthalten, die auf einem oder mehreren Kollagenen, Fibrin oder Fibrinderivaten, Hyaluronsäurederivaten oder Hydrogelen oder Gemischen davon basieren.

Im Rahmen der der vorliegenden Erfindung zugrunde liegenden Studien wurde gefunden, daß insbesondere autologe Hautzelltransplantate, welche kultivierte autologe Melanozyten alleine oder in Verbindung mit Keratinozyten in einer Trägermatrix, welche eine formbare, gelartige Konsistenz aufweist, enthalten, im Tierexperiment und bei Vitiligopatienten ausgesprochen gute Transplantationseigenschaften aufweisen. Es sind insbesondere dann gute Therapieerfolge zu beobachten, wenn das von Vitiligo betroffene Empfängerareal einer geeigneten Vorbehandlung unterzogen wird, insbesondere wenn es beispielsweise vor der Transplantation mit Er-Yag Laser abgeschliffen wird. Anschließen werden die erfindungsgemäß kultivierten und in eine geeignete Trägermatrix, etwa Fibrinkleber, aufgenommene Melanozyten auf dieses Areal aufgetragen, welches dann verbunden wird. In der Regel kann der Primärverband nach etwa einer Woche entfernt werden, die transplantierten Stellen werden bevorzugt etwa einmal wöchentlich UV-bestrahlt. Hierbei ist prinzipiell etwa eine UVB- oder eine UVA-Bestrahlung, beispielsweise im Rahmen einer PUVA-Therapie, möglich. Eine bevorzugte Ausfühungsform beinhaltet eine UVB-Bestrahlung einer Wellenlänge von 311 nm und geeigneter Dauer.

Es versteht sich von selbst, daß eine Kombination mit weiteren möglichen sinnvollen Therapiemaßnahmen nicht ausgeschlossen wird. Bei einer Therapie der beispielhaft genannten Art lassen sich häufig bereits nach einigen Wochen die ersten Repigmentierungsvorgänge beobachten.

Speziell im Zusammenhang mit der Transplantation kultivierter, insbesondere autologer Melanozyten und/oder Keratinozyten ist - neben der bereits erläuterten Vitiligotherapie - des weiteren eine Verwendung im Rahmen einer Repigmentierung bzw. einer Imitation von Brustwarzen oder einem Brustaufbau denkbar, beispielsweise nach einer totalen oder subtotalen Brustentfernung. Hierzu können kultivierte, insbesondere autologe Melanozyten alleine oder in Verbindung mit anderen, in der Regel ebenfalls kultivierten Zelltypen, etwa Keratinozyten, Fibroblasten, Chondrozyten, Nervenzellen sowie Gefäßzellen verwendet werden. Im Falle eines Brustaufbaus wird dies natürlich sinnvollerweise im Zusammenhang bzw. in. Kombination mit dem Fachmann bekannten sonstigen Methoden durchgeführt werden, etwa einer freien Gewebeverpflanzung oder einem Aufbau mit Silikonimplantaten.

Hautzelltransplantate, die gemäß dem erfindungsgemäßen Verfahren hergestellt werden, zeichnen sich nicht nur durch die generellen, bereits genannten Vorteile solcher auf autologen Zellen basierender Transplantate aus, sondern weisen darüber hinaus noch zusätzliche Vorteile auf.

In diesem Zusammenhang ist insbesondere noch einmal darauf hinzuweisen, daß die formbare, gelartige Konsistenz der im Rahmen dieser Erfindung bevorzugten

Transplantate besonders geeignet für eine Auftragung an Stellen wie etwa den Händen, dem Nacken, dem Hals sowie dem Genitalbereich, deren Behandlung mit den im Stand der Technik beschriebenen Methoden problematisch war. Dies sind jedoch diejenigen Stellen, an denen Vitiligo in der Regel am häufigsten auftritt. Durch die Formbarkeit werden die Zellen effektiver transplantiert, und ihre anschließende Adhäsion, Proliferation und Migration wird gefördert, was wiederum zu einem besseren "Take", d.h. einer besseren Integration des Transplantats, fühlt. Prinzipiell gilt, daß je größer der "Take", desto größer auch die Chance für eine optimale Repigmnentierung.

Zusammenfassend läßt sich somit feststellen, daß Hautzelltransplantationen in Zusammenhang mit den hierin beschriebenen Verfahren, beispielsweise, aber keineswegs ausschließlich im Rahmen der Vitiligotherapie, nicht nur viel einfacher im Vergleich mit anderen Methoden durchzuführen sind, sondern darüber hinaus auch den Erfolg der Transplantation und der Repigmentierung signifikant fördern.

Schließlich wurde auch gefunden, daß autologe Hautzellentransplantate, welche kultivierte autologe Melanozyten und/oder Keratinozyten in einer Trägermatrix, welche eine formbare, gelartige Konsistenz aufweist, enthalten, auch sehr gute Transport- und Applikationseigenschaften aufweisen. So wurde beispielsweise gezeigt, daß autologe Melanozyten in einer bevorzugten Ausführungsform der erfindungsgemäßen, nachfolgend näher beschriebenen Zusammensetzungen selbst nach einem Transport oder einer Aufbewahrung bei Raumtemperatur für 24 Stunden in aller Regel noch eine Vitalität von bis zu 80% zeigen.

Eine geeignete Trägermatrix kann prinzipiell entweder auf Biomolekülen basieren, die *in vivo* beispielsweise als Bestandteile der extrazellulären Matrix (ECM) vorkommen, etwa Kollagene, Hyaluronsäureester und Glykosaminoglykansäureester, oder auf Derivaten solcher ECM-Moleküle, des weiteren auf Fibrin oder Fibrinderivaten sowie auf geeigneten synthetischen Materialien. In der Regel werden biologisch abbaubare Materialien ("Biomatrizes") bevorzugt, besonders bevorzugt sind Biomatrizes auf der Basis von Fibrinkleber und/oder anderen Fibrinderivaten.

Fibrinkleber stellen biologische Zweikomponenten-Trägersysteme dar, die im wesentlichen aus einer Fibrinogen- und einer Thrombinkomponente bestehen. Durch die Zugabe der Thrombinkomponente wird die Koagulierung, d.h. die Umsetzung vom Fibrinogen zu Fibrin, induziert. Der Koagulationsgrad und somit die Konsistenz des Trägersystems läßt sich anhand der gewählten Thrombinkonzentration einstellen. Eine detaillierte Beschreibung des Trägersystems Fibrinkleber, seiner Herstellung, seine Verwendung fur die Kultur von Keratinozyten sowie die Verwendung solcher Keratinozytenkulturen als Transplantate enthält die EP 0 373 044, deren diesbezüglicher Inhalt vollumfänglich in die vorliegende Anmeldung einbezogen wird.

Im Rahmen der der vorliegenden Erfindung zugrunde liegenden Studien wurde gezeigt, daß Hautzellen, insbesondere Melanozyten und/oder Keratinozyten, die in Form eines nach dem erfindungsgemäßen verfahren hergestellten Transplantats transplantiert werden, nach einer autologen Transplantation schnell an das entsprechend vorbehandelte Gewebe des Empfängerareals adhärieren, proliferieren und migrieren. Dadurch werden im Vergleich mit den herkömmlich in der Vitiligotherapie verwendeten Melanozytentransplantaten häufig schnellere Therapieerfolge erzielt, ohne daß die Qualität oder Dauerhaftigkeit der Repigmentierung oder das kosmetische Ergebnis, etwa durch Narbenbildung, beeinträchtigt werden.

Die Transplantation erfolgt hierbei nach dem Fachmann bekannten Methoden, die gegebenenfalls eine Vor- und/oder Nachbehandlung des zu behandelnden Areals, etwa mittels einer Laservorbehandlung zur Abschleifung des Gewebes, etwa mittels eines ER-YAG-Lasers, und/oder einer UV-Bestrahlung, umfassen können.

Eine für diesen Zweck geeignete Trägermatrix kann prinzipiell entweder auf biologischen Molekülen basieren, die *in vivo* beispielsweise als Bestandteile der extrazellulären Matrix (ECM) vorkommen, etwa Kollagene, Hyaluronsäureester und Glykosaminoglykansäureester, oder auf Derivaten solcher ECM-Moleküle, des weiteren auf Fibrin oder Fibrinderivaten sowie auf geeigneten synthetischen Trägermaterialien. In der Regel werden biologisch abbaubare Materialien bevorzugt, d.h., es werden wiederum bevorzugt Biomatrizes eingesetzt, wobei Biomatrizes auf der Basis von Fibrinkleber und/oder anderen Fibrinderivaten besonders bevorzugt sind.

Der Fachmann wird nach den obigen Ausführungen verstehen, daß auch in diesem Fall die Kultivierung der Hautzellen, d.h. der Keratinozyten alleine oder in Verbindung mit anderen Zelltypen, prinzipiell gemäß allen dem Fachmann bekannten eukaryotischen Zellkultivierungsverfahren, einschließlich des hier offenbarten erfindungsgemäßen Kultivierungsverfahrens, erfolgen kann.

Die Transplantation erfolgt gemäß dem Fachmann bekannten Methoden, die gegebenenfalls eine Vor- und/oder Nachbehandlung des zu behandelnden Areals, etwa mittels einer Laservorbehandlung zur Abschleifung des Gewebes, etwa mittels eines ER-YAG-Lasers, und/oder einer UVB-Bestrahlungsnachbehandlung umfassen können.

Im folgenden soll nunmehr die Erfindung anhand eines Ausführungsbeispiels näher erläutert, aber keinesfalls beschränkt werden.

### Beispiel:

Eine Biopsie, die einem Patienten entnommen wurde, wird nach Desinfektion in eine Umgebung gebracht, in der die Bedingungen der Reinheitsklasse A gemäß dem EG-Leitfaden einer Guten Herstellungspraxis für Arzneimittel eingehalten werden, beispielsweise durch einen Transferisolator in einen Arbeitsisolator, wobei jeweils nahezu absolute Keimfreiheit herrscht.

Im Falle einer Melanozytenkultivierung werden folgende Schritte, bevorzugt unter den jeweils in Klammem genannten Reinheitsbedingungen, durchgeführt:
- Präparation der Hautbiopsie, mechanische Entfernung von Dermisresten (A)
- Spülung mit 70%-igem Ethanol und dann mit PBS-Puffer (A)
- Enzymatischer Verdau zur Ablösung der Epidermis von der Dermis (mindestens 2,4 U/ml Dyspase, entweder 4 Stunden bei 37°C oder über Nacht bei 4°C) (D)
- Trennung der Epidermis von der Dermis durch "Abziehen" (A)
- Zerkleinerung der Epidermis in einer PBS-Pufferlösung mittels sterilen Instrumente, beispielsweise Scheren oder Pinzetten (A)
- Enzymatische Dissoziation einzelner Zellen durch Trypsinierung (0,5%-ige Trypsinlösung; 30 Minuten bei 37°C) (A oder D)
- Stoppen der Trypsinierung, beispielsweise durch Zugabe von Serum (A)
- Zentrifugation (zwischen 1500 und 4000 upm; bei Raumtemperatur; etwa 5 bis 10 Minuten) (D)
- Absaugen des Überstandes und Resuspension der Zellen in einem geeigneten Melanozytennährmedium (z.B. in M2-Medium der Fa. PromoCell, Heidelberg) (A)
- Aussäen der Zellen in Kulturflaschen (5000 - 50000 Zellen/cm²) (A)
- Kultivierung der Zellen bis zur Subkonfluenz (37°C, 5% CO₂ Mediumwechsel nach Bedarf, in der Regel jeden zweiten oder dritten Tag) (D)

Fertigstellung eines autologen Melanozytentransplantats:
- Enzymatische Dissoziation einzelner Zellen durch Trypsinierung (0,5%-ige Trypsinlösung; 30 Minuten bei 37°C) (A oder D)
- Stoppen der Trypsinierung, beispielsweise durch Zugabe von Serum (A)
- Zentrifugation (zwischen 1500 und 4000 upm; bei Raumtemperatur; etwa 5 bis 10 Minuten) (D)
- Resuspension der Zellen und Aufnahme der Zellen in Thrombinlösung (4 Mio Zellen /0,5 ml (A) oder in an anderes geeignetes Matrixmaterial.

Nicht in Thrombinlösung aufgenommene Zellen werden entweder sofort oder nach erneuter Weiterkultivierung bis zur Subkonfluenz kryokonserviert.

Vor der Auftragung des autologen Transplantats wird das Vitiligoareal mit Er-Yag Laser abgeschliffen. Auf die entsprechenden Stellen wird die Suspension der autologen kultivierten Melanozyten in Fibrinkleber aufgetragen. Die Stellen werden verbunden, der Primärverband wird nach einer Woche entfernt. Danach erfolgt wöchentlich eine UV-Bestrahlung (UVB, 311nm, über einen Zeitraum von üblicherweise mindestens 12 Wochen). Üblicherweise setzt nach einigen Wochen eine Repigmentierung der behandelten Hautbereiche ein.

## Patentansprüche

1. Verfahren zur Herstellung eines humanen autologen Transplantats, **dadurch gekennzeichnet, dass**
(1) humane eukaryotische Zellen so kultiviert werden, dass Schritte, bei denen die Zellkulturen offen sind, in einem geschlossenen System bei Überdruck durchgeführt werden, und dass in dem System
(a) pro Kubikmeter Luft nicht mehr als 3500 Partikel vorhanden sind, die mindestens 0,5 µm groß sind; und
(b) pro Kubikmeter Luft keine Partikel vorhanden sind, die mindestens 5 µm groß sind;
(2) die kultivierten Zellen unter den in (1) genannten Bedingungen in eine geeignete Matrix aufgenommen werden,
wobei das Transplantat ein dreidimensionales Transplantat ist, innerhalb dessen die kultivierten autologen Zellen in Verbindung mit einer geeigneten Matrix in eine vorgegebene, dreidimensionale Form gebracht wurden.

2. Verfahren nach Anspruch 1, **dadurch gekennzeichnet, dass** in (1) Hautzellen, Knochenzellen, Knorpelzellen, Fettzellen, Gefäßzellen, Mundschleimhautzellen, Urothelzellen, Muskelzellen, Nervenzellen, hämatopoetische Zellen, Sehnenzellen, andere Zellen des Bewegungssystems, Stammzellen mit Ausnahme embryonaler Stammzellen, Leberzellen, Nierenzellen, Herzmuskelzellen, verschiedene Schleimhautepfthelzellen sowie Hormone oder Transmitter produzierende Zellen kultiviert werden.

3. Verfahren nach Anspruch 1 oder 2, **dadurch gekennzeichnet, dass** in (1) Hautzellen, Knorpelzellen und / oder Knochenzellen kultiviert und in eine Matrix aufgenommen werden, welche auf biologischen Molekülen, insbesondere auf extrazellulären Matrixmolekülen oder Derivaten davon, oder auf synthetischen Molekülen basiert.

4. Verfahren nach Anspruch 3, **dadurch gekennzeichnet, dass** die Hautzellen Melanozyten und / oder Keratinozyten sind.

5. Verfahren nach Anspruch 3 oder 4, **dadurch gekennzeichnet, dass** eine Matrix eingesetzt wird, die eine formbare oder gelartige Konsistenz aufweist.

6. Verfahren nach Anspruch 4 oder 5, **dadurch gekennzeichnet, dass** zur Herstellung des Transplantats eine Matrix eingesetzt wird, die Kollagengel, Fibrinkleber oder ein oder mehrere andere Fibrinderivate, Hyaluronsäurederivate oder Hydrogele oder Mischungen davon enthält.

## Claims

1. Method for producing a human autologous transplant, **characterized in that**,
(1) human eukaryotic cells are cultured such, that steps during which the cell cultures are exposed, are conducted in a closed system with an overpressure, and that in said system
(a) per cubic meter of air, not more than 3500 particles are present, which are at least 0.5 µm in size, and
(b) per cubic meter of air, no particles are present, which are at least 5 µm in size;
(2) the cultured cells under the conditions outlined above in (1), are
incorporated into a suitable matrix,
wherein the transplant is a three-dimensional transplant, in which the cultured autologous cells in combination with a suitable matrix, were brought into a predetermined, three-dimensional shape.

2. Method according to claim 1, **characterized in that** in (1), skin cells, bone cells, cartilage cells, fat cells, vascular cells, buccal cells, urothelial cells, muscle cells, nerve cells, hematopoietic cells, tendon cells, other cells of the musculoskeletal system, stem cells other than embryonic stem cells, liver cells, kidney cells, heart muscle cells, different mucous epithelial cells, as well as cells producing hormones or transmitters, are cultured.

3. Method according to claim 1 or 2, **characterized in that** in (1), skin cells, cartilage cells, and/or bone cells are cultured and incorporated into a matrix, which is based on biological molecules, in particular, on extracellular matrix molecules, or derivatives thereof, or which is based on synthetic molecules.

4. Method according to claim 3, **characterized in that**, the skin cells are melanocytes and/or keratinocytes.

5. Method according to claim 3 or 4, **characterized in that** a matrix is used, which has a shapeable or gel-like consistency.

6. Method according to claim 4 or 5, **characterized in that**, for producing the transplant, a matrix is used, which contains collagen gel, fibrin glue, or one or several other fibrin derivative(s), hyaluronic acid derivatives, or hydrogels, or mixtures thereof.

## Revendications

1. Procédé de préparation d'un transplant humain autologue, **caractérisé en ce que**
(1) des cellules eucaryotes humaines sont cultivées de telle sorte que les étapes dans lesquelles les cultures cellulaires sont ouvertes sont effectuées dans un système fermé en surpression, et **en ce que**
(a) par mètre cube d'air, il n'y a pas plus de 3500 particules d'une taille d'au moins 0,5 µm, et que
(b) par mètre cube d'air, il n'y a pas de particules qui aient au moins 5 µm de taille,
(2) **en ce que** les cellules cultivées sous les conditions énoncées en (1) sont logées dans une matrice appropriée,
sachant que le transplant est un transplant tridimensionnel, à l'intérieur duquel les cellules autologues cultivées sont amenées dans une forme tridimensionnelle prédéfinie en relation avec une matrice appropriée.

2. Procédé selon la revendication 1, **caractérisé en ce qu'**en (1) sont cultivées des cellules cutanées, des cellules osseuses, des cellules cartilagineuses, des cellules adipeuses, des cellules vasculaires, des cellules muqueuses buccales, des cellules urothéliques, des cellules musculaires, des cellules nerveuses, des cellules hématopoïétiques, des cellules tendineuses, d'autres cellules du système locomoteur, des cellules souches à l'exception des cellules souches embryonnaires, des cellules hépatiques, des cellules rénales, des cellules myocardiques, différentes cellules épithéliales muqueuses, ainsi que des cellules produisant des hormones ou des transmetteurs.

3. Procédé selon la revendication 1 ou 2, **caractérisé en ce que** des cellules cutanées, des cellules cartilagineuses et/ou des cellules osseuses sont cultivées en (1) et sont logées dans une matrice qui se base sur des molécules biologiques, en particulier sur des molécules de matrice extracellulaire ou sur des dérivés de celles-ci, ou sur des molécules synthétiques.

4. Procédé selon la revendication 3, **caractérisé en ce que** les cellules cutanées sont des mélanocytes et/ou des kératinocytes.

5. Procédé selon la revendication 3 ou 4, **caractérisé en ce que** l'on utilise une matrice qui présente une consistance moulable ou géliforme.

6. Procédé selon la revendication 4 ou 5, **caractérisé en ce que** pour la préparation du transplant, on utilise une matrice qui contient un gel de collagène, une colle de fibrine ou un ou plusieurs autres dérivés de la fibrine, des dérivés de l'acide hyaluronique ou des hydrogels ou des mélanges de ceux-ci.
